# EUROPEAN PATENT APPLICATION

(11) **EP 1 074 217 A1**
(43) Date of publication of application: **07.02.2001**
(21) Application number: 00306340.1
(22) Date of filing: 26.07.2000
(51) Int. Cl.: A61B 5/103

(54) **Floor reaction force measuring method and apparatus therefor**

(30) Priority: 05.08.1999 JP 22277599
(71) Applicant: Anima Corporation, Tokyo 160-0023 (JP)
(72) Inventor: Okuda, Toshihito, Tokyo 160-0023 (JP); Murase, Hitoshi, Tokyo 160-0023 (JP); Nakajima, Takao, Tokyo 160-0023 (JP)
(74) Representative: Ajello, Michael John

(57) **Abstract**

A measuring apparatus for measuring reaction forces exerted by a subject against a support surface. The measuring apparatus comprising: a forceplate (10) comprising a platform (11) with the support surface for placing the subject thereon, and a reaction force measuring member (13) disposed under the platform, for sensing the reaction forces exerted by the subject against the platform; a distribution shape sensing sheet (15) for sensing a shape of distribution of the reaction forces exerted by the subject against the platform, or a distribution pressure measuring sheet for sensing distribution pressure of the reaction forces, which is placed on an upper surface of the platform (11); an output member for outputting analysis data on the basis of data of the reaction forces sensed by the reaction force measuring member and data of the shape of distribution sensed by the distribution shape sensing sheet or of the distribution pressure sensed by the distribution pressure measuring sheet.

## Description

### BACKGROUND OF THE INVENTION

### Field of the Invention

The present invention relates to a measuring method for measuring reaction forces exerted by the foot or feet of a subject such as a human, an animal or the like, against a support surface, and an apparatus for embodying the method. Such a measuring method or apparatus is used for testing the subject's sway during erect standing, or for analyzing the subject's balancing function, motility or the like, during moving, e.g., walking, stepping up and down, and the like, in the fields of the body sway test, the orthopedic surgery, the rehabilitation, the biomechanical science or the like.

In the test of balancing function which is a principal matter for an examination of dizziness or equilibrium disorder, or in the field of the rehabilitation, the physical training or sports medical science or the like, a stabilometer is used for recording the movement or sway of the body during erect standing as a sway of the body center of gravity (pressure), and then analyzing to seize the features, characteristics, direction and the like, of the pattern of the sway on the basis of the measured data. The analyzed results facilitate an examination of the focus to the balancing function, the nerve function or the like, and facilitate an extremely objective judgment and seizing the recovery condition of the balancing function, the nerve function or the like by rehabilitation.

In such an examination of balancing function or the like using a stabilometer, two measurements of the sway of the subject's body center of pressure are carried out under two different conditions of the eyes opening and closing.

In the measurement using a stabilometer, the load information by the subject with keeping an erect standing posture on the forceplate is measured by a plurality of load detection sensors (load cells). The position of the center (sway center) of reaction force exerted by the subject is calculated on the basis of the respective load information measured by the plurality of load cells.

The stabilometer measures the vertical reaction force by the subject against the forceplate, by using a plurality of load cells. A floor reaction force measuring apparatus for measuring reaction forces to a support surface is also known as an apparatus which is used in the field of the rehabilitation medical science, the physical training or sports medical science or the like.

In floor reaction force measuring apparatus for measuring reaction forces to a support surface, for example, a known three-force components detecting load cell is used instead of the load cell for measuring the load of the vertical reaction force. Each three-force components detecting load cell comprises X and Y force component detectors which detect force components along the X and Y axes which are perpendicular to each other, of the reaction force exerted to a platform of a forceplate, on the upper surface of the platform, respectively, and a Z force component detector which detects the force component along the Z-axis which is perpendicular to the upper surface of a platform, of the reaction force exerted to the platform.

Such a reaction force measuring apparatus for measuring reaction forces to a support surface, having three-force components detecting load cells, enables measurement of the subject's weight distribution during taking exercise, acceleration force and decceleration force, locomotion of weight, torsion force, step length during walking, walking speed and the like, according to the detection results from each of the X, Y and Z force component detectors of the three-force components detecting load cells and the information for the moment about each axis.

A thin distribution shape sensing sheet to be set on a floor, for detecting a walking pattern is known.

Recently, a pressure distribution sensing sheet for sensing the walking pattern of foot and the magnitude of foot pressure in real time, is also known.

Such a pressure distribution sensing sheet enables measurement of various types of time parameters, e.g., single support time, double support time, stance phase time, walking cycle and the like, distance parameters, e.g., stride length, step length, step width, toe out angle and the like, and floor reaction force against the supporting surface, i.e., gravity component, by setting it on the supporting surface and by a subject walking thereon.

According to use of a distribution shape sensing sheet using a pressure sensitive resistive material layer, piezo-electric sensors or the like, although it is possible to obtain a shape of distribution of the load exerted to the upper surface of the platform, it is not possible to know the information about the magnitude of the load nor the position of the center of pressure.

According to use of a pressure distribution sensing sheet, it is possible to provide a measurement and display of a shape of distribution of the load exerted to the upper surface of the platform and the position of the center of pressure, at the same time. However, it is not possible to provide an accurate measurement nor accurate display of the position of the center of pressure because such a pressure distribution sensing sheet has a non-linear force/resistance characteristics. Further, such a sheet has a large problem for the measuring apparatus in which a human body contacts with the sheet directly, that the output thereof depends on the temperature, that is, the subject's temperature leads to an error.

Further, use of a pressure distribution sensing sheet has a large problem that when using the one placed on an inclined surface, a large error for load occurs because the direction of force by a subject is not perpendicular to the upper surface of the pressure distribution sensing sheet.

Therefore, the conventional measurement apparatus using such a pressure distribution sensing sheet could not provide a high-accuracy measurement result.

The measurement apparatus using a forceplate with load cells, no distribution shape sensing sheet and no pressure distribution sensing sheet, has a problem that it cannot provide a shape of distribution of the load exerted to the upper surface of the platform.

### SUMMARY OF THE INVENTION

The invention has been made in view of the above problems.

An object of the invention is to provide a measuring apparatus and a method, for measuring reaction forces exerted by a subject against a support surface, which enables high-accuracy measurement of the magnitude of the load, the position of the center of pressure and a shape of distribution of the load exerted to the upper surface of the platform, at the same time.

In accordance with an aspect of the invention, the measuring apparatus for measuring reaction forces exerted by a subject against a support surface, comprising:
a forceplate (10, 20 or 30) comprising a platform (11, 21 or 31) with the support surface for placing the subject thereon, and a reaction force measuring member (13, 23 or 33) disposed under the platform, for sensing the reaction forces exerted by the subject against the platform,
a distribution shape sensing sheet (15, 25 or 35) for sensing a shape of distribution of the reaction forces exerted by the subject against the platform, or a distribution pressure measuring sheet for sensing distribution pressure of the reaction forces, which is placed on an upper surface of the platform, and
an output member (45 or 46) for outputting analysis data on the basis of data of the reaction forces sensed by the reaction force measuring member and data of the shape of distribution sensed by the distribution shape sensing sheet or of the distribution pressure sensed by the distribution pressure measuring sheet.

The measuring apparatus for measuring reaction forces has the function of a stabilometer, a floor reaction force measuring apparatus, and an imager of distribution shape of the load exerted against the surface.

The forceplate is used mainly as a stabilometer and a surface reaction force measuring apparatus.

As the reaction force measuring member, preferably, three or four load cells may be used for obtaining the function of stabilometer, and four three-force components detecting load cells may be used for obtaining the function of the surface reaction force measuring apparatus. The number and the type of load cells to be used can be selected optionally.

As the distribution shape sensing sheet, one of various types of known sheet-like matrix-arrayed pressure sensors can be adopted. For example, it may comprise a known structure in which a matrix array of pressure sensitive elements, e.g., resistive or inductive force sensing elements, piezo-electric elements or the like, is sandwiched between a large number of row electrodes which are formed on the inner surface of a first film substrate and a large number of column electrodes which are formed on the inner surface of a second film substrate, wherein the row and column electrodes form a matrix array of crossover points and have electrode terminals at the respective peripheral ends.

The output member includes a printing device, a display device or the like.

According to the measuring apparatus for measuring reaction forces of the invention, it is possible not only to detect a shape of distribution of the load or to sense distribution pressure of the reaction forces, exerted to the upper surface of the platform, by a distribution shape sensing sheet or a distribution pressure measuring sheet which is placed on an upper surface of the platform, but also to measure the magnitude of the load and the position of the center of pressure highly accurately by the load cells disposed under the platform at the same time because the load cells have a linear force/resistance characteristics and the output thereof is not affected by the subject's temperature.

The measuring apparatus may further comprise a supplementary support having stairs, an upper inclined surface or an upper curved surface, on an upper surface of which the distribution shape sensing sheet or the distribution pressure measuring sheet is placed.

According to the measuring apparatus having such a structure, it is possible to obtain all of dynamic analysis data, i.e., dynamic loads (force), dynamic sway of the subject's body center of pressure (COP), and dynamic distribution shapes by the foot or feet, during ascending or descending the stairs, on the upper inclined surface or on the upper curved surface, extremely accurately at the same time.

Even when using a pressure distribution sensing sheet placed on an inclined surface, it is possible to measure the information for load, e.g., the sway of subject's COP very accurately because the load cells have a linear force/resistance characteristics and the output thereof is not affected by the subject's temperature.

In accordance with another aspect of the invention, the measuring apparatus for measuring reaction forces exerted by a subject against a support surface, comprising:
a walkway comprising a set of a plurality of gait plates arranged in order, each of the steps comprising: a forceplate (10, 20 or 30) having a platform (11, 21 or 31) with the support surface for placing the subject thereon, and a reaction force measuring member (13, 23 or 33) disposed under the platform, for sensing the reaction forces exerted by the subject against the platform; and a distribution shape sensing sheet (15, 25 or 35) for sensing a shape of distribution of the reaction forces exerted by the subject against the platform, or a distribution pressure measuring sheet for sensing distribution pressure of the reaction forces, which is placed on an upper surface of the platform; and
an output member (45 or 46) for outputting analysis data on the basis of data of the reaction forces sensed by the reaction force measuring member and data of the shape of distribution sensed by the distribution shape sensing sheet or of the distribution pressure sensed by the distribution pressure measuring sheet, of the gait plates.

According to the measuring apparatus having such a structure, it is possible to obtain all of dynamic analysis data, i.e., dynamic loads (force), dynamic positions of the subject's body center of pressure, and dynamic distribution shapes or distribution pressure of the reaction forces by the foot or feet, during moving on the walkway, extremely accurately at the same time because the load cells have a linear force/resistance characteristics and the output thereof is not affected by the subject's temperature.

In accordance with another aspect of the invention, the measuring apparatus for measuring reaction forces exerted by a subject against a support surface, comprises:
a supplementary support (50 or 60) which comprises: a forceplate (10, 20 or 30) having a rigid platform and three or more load cells (13, 23 or 33) provided under the platform at peripheral corners thereof to support the platform; a body having at least one of stairs, a slope and a curved surface, placed on the upper surface of the platform; and a distribution shape sensing sheet (55 or 65) for sensing a shape of distribution of the reaction forces exerted by the subject against the platform, or a distribution pressure measuring sheet for sensing distribution pressure of the reaction forces, which is placed on an upper surface of the body, and
an output member (45 or 46) for outputting analysis data on the basis of data of the reaction forces sensed by the load cells and data of the shape of distribution sensed by the distribution shape sensing sheet or of the distribution pressure sensed by the distribution pressure measuring sheet.

According to the measuring apparatus having such a structure, it is possible to obtain the dynamic analysis data, i.e., dynamic loads (force), dynamic positions of the subject's body center of pressure, and dynamic distribution shapes or distribution pressure of the reaction forces by the foot or feet, during ascending or descending the stairs, on the upper inclined surface or on the upper curved surface, extremely accurately at the same time.

In particular, even when using the distribution shape sensing sheet placed on an inclined surface, the dynamic load exerted by the subject against the platform can be measured accurately by the load cells under the platform, in spite the direction of force by a subject being not perpendicular to the upper surface of the pressure distribution sensing sheet because each load cell has a linear force/resistance characteristics and the output thereof is not affected by the subject's temperature.

The output member may be a display device which displays: a shape of distribution of the reaction forces on the basis of the data sensed by the distribution shape sensing sheet or a state of distribution pressure on the distribution pressure measuring sheet on the basis of data sensed by the distribution pressure measuring sheet; and a center of pressure on the basis of data analysis of the reaction forces sensed by the reaction force measuring member.

In accordance with another aspect of the invention, the measuring apparatus for measuring reaction forces exerted by a subject against a support surface, comprising:
a forceplate comprising a rigid platform with the support surface for placing the subject thereon, and a plurality of reaction force measuring members disposed at peripheral positions of and under the platform, for detecting loads exerted to the respective disposed positions on the platform continuously and outputting load information signals on the basis of the detected loads,
a distribution shape sensing sheet for sensing a shape of distribution of the reaction forces exerted by the subject against the platform, or a distribution pressure measuring sheet for sensing distribution pressure of the reaction forces, which is placed on an upper surface of the platform,
a processor for calculating to determine a total amount of reaction force exerted to the platform by the subject, a position of the center of reaction force and a shape of distribution or distribution pressure, of the reaction force, on the basis of load information signals from the reaction force measuring members and shape of distribution signals from the distribution shape sensing sheet or distribution pressure signals from the distribution pressure measuring sheet, respectively, and
an output member to enable outputting the total amount of reaction force exerted to the platform by the subject, the position of the center of reaction force and the shape of distribution or the distribution pressure, of the reaction force, determined by the processor.

Preferably, the plurality of reaction force measuring members comprise load cells or three-force components detecting load cells, not less than 3.

In accordance with another aspect of the invention, the measuring method for measuring reaction forces exerted by a subject against a support surface, comprising the steps of:
preparing a forceplate comprising a rigid platform with the support surface for thereon, and a plurality of reaction force measuring members disposed at peripheral positions of and under the platform, to detect loads exerted to the respective disposed positions on the platform continuously and to output load information signals on the basis of the detected loads,
placing a distribution shape sensing sheet for sensing a shape of distribution of the reaction forces or a distribution pressure measuring sheet for measuring distribution pressure of the reaction forces on an upper surface of the platform,
placing a subject on an upper surface of the distribution shape sensing sheet or the distribution pressure measuring sheet,
calculating to determine a total amount of reaction force exerted to the platform by the subject, a position of the center of reaction force and a shape of distribution of the reaction force or distribution pressure of the reaction force, by a processor, on the basis of load information signals from the reaction force measuring members and shape of distribution signals from the distribution shape sensing sheet or distribution pressure signals from the distribution pressure measuring sheet, respectively, and
outputting the total amount of reaction force, the position of the center of reaction force exerted to the platform by the subject and the shape of distribution or the distribution pressure, of the reaction force, determined by the processor.

According to the measuring method for measuring reaction forces of the invention, because sensing the shape of distribution or distribution pressure, of the reaction forces is performed by a distribution shape sensing sheet or a distribution pressure measuring sheet, and measurement of the magnitude of the load and the position of the center of pressure are independently performed by a plurality of reaction force measuring members disposed at peripheral positions of and under the platform and because the load cells have a linear force/resistance characteristics and the output thereof is not affected by the subject's temperature, it is possible to obtain the analysis data, i.e., loads (force), positions of the subject's body center of pressure, and distribution shapes or distribution pressure of the reaction forces by the foot or feet, extremely accurately at the same time.

Preferably, the step of outputting comprises displaying the shape of distribution of the reaction forces on the basis of the data sensed by the distribution shape sensing sheet or a state of distribution pressure on the distribution pressure measuring sheet on the basis of data sensed by the distribution pressure measuring sheet; and displaying the center of pressure on the basis of data analysis of the reaction forces sensed by the reaction force measuring member, at the same time.

In accordance with another aspect of the invention, the measuring method for measuring reaction forces exerted by a subject against a support surface, comprising the steps of:
placing the subject on a walkway comprising a set of a plurality of gait plates arranged in order, each of the gait plates comprising: a forceplate having a platform with the support surface for placing the subject thereon, and a reaction force measuring member disposed under the platform, for sensing the reaction forces exerted by the subject against the platform; and a distribution shape sensing sheet for sensing a shape of distribution of the reaction forces exerted by the subject against the platform, or a distribution pressure measuring sheet for sensing distribution pressure of the reaction forces, which is placed on an upper surface of the platform; and
sensing a shape of distribution of the reaction forces exerted by the subject against the platform, by using a distribution shape sensing sheets of the gait plates, or sensing distribution pressure of the reaction forces by using a distribution pressure measuring sheet of the gait plates, which are placed on upper surfaces of the platforms of the gait plates,
calculating to determine a total amount of reaction force exerted to each platform by the subject, a position of the center of reaction force and shapes of distribution of the reaction force or distribution pressure of the reaction force, by a processor, on the basis of load information signals from the reaction force measuring members and shape of distribution signals from the distribution shape sensing sheets or distribution pressure signals from the distribution pressure measuring sheets, respectively, and
outputting the total amount of reaction force, the position of the center of reaction force exerted to the platform by the subject and the shape of distribution or the distribution pressure, of the reaction force, on each gait plate, determined by the processor.

The step of outputting may comprise:
displaying a locus of shape of distribution of the reaction forces on the basis of the data sensed by the distribution shape sensing sheets of the gait plates or a locus of a state of distribution pressure on the distribution pressure measuring sheets on the basis of data sensed by the distribution pressure measuring sheets; and a locus of a center of pressure on the basis of data analysis of the reaction forces sensed by the reaction force measuring members, at the same time.

According to the measuring method having such steps, it is possible to obtain all of dynamic analysis data, i.e., dynamic loads (force), dynamic positions of the subject's body center of pressure, and dynamic distribution shapes or distribution pressure of the reaction forces by the foot or feet, during moving on the walkway, extremely accurately at the same time because of use of the load cells having a linear force/resistance characteristics and the output thereof being not affected by the subject's temperature.

### BRIEF DESCRIPTION OF THE DRAWINGS

The present invention will become more fully understood from the detailed description given hereinbelow and the accompanying drawings which are given by way of illustration only, and thus are not intended as a definition of the limits of the present invention, and wherein;
FIG. 1 is a perspective view of a first embodiment of a forceplate having a distribution shape sensing sheet, which is used in the measuring apparatus according to the present invention;
FIG. 2 is a perspective view of an embodiment of a walkway which is formed by a plurality of forceplates each having a distribution shape sensing sheet which are shown in FIG. 1;
FIG. 3 is a block diagram of the measuring apparatus using the walkway shown in FIG. 2;
FIG. 4 is a perspective view of a second embodiment of a forceplate having a distribution shape sensing sheet, which is used in the measuring apparatus according to the present invention;
FIG. 5 is a perspective view of an embodiment of a walkway which is formed by a plurality of forceplates each having a distribution shape sensing sheet which are shown in FIG. 4;
FIG. 6 is a block diagram of the measuring apparatus using the walkway shown in FIG. 5;
FIG. 7 is a perspective view of a third embodiment of a forceplate having a distribution shape sensing sheet, which is used in the measuring apparatus according to the present invention;
FIG. 8 is a perspective view of an embodiment of a walkway which is formed by a plurality of forceplates each having a distribution shape sensing sheet which are shown in FIG. 7;
FIG. 9 is a block diagram of the measuring apparatus using the walkway shown in FIG. 8;
FIG. 10 is a view showing footprints with distribution shape and a locus of the center of pressure, as an output example of analysis data for gait patterns during walking by the measuring apparatus shown in FIG. 9;
FIGS. 11A and 11B are views showing distance parameters with reference to right foot and left foot, respectively, as output examples of analysis data for footfall patterns by the measuring apparatus shown in FIG. 9;
FIG. 12 is a perspective view of the main part of the measuring apparatus according to a fourth embodiment of the present invention;
FIG. 13 is a perspective view of the main part of the measuring apparatus according to a fifth embodiment of the present invention;
FIG. 14 is a perspective view of the main part of the measuring apparatus according to a sixth embodiment of the present invention;
FIG. 15 is a perspective view of the main part of the measuring apparatus according to a seventh embodiment of the present invention;
FIG. 16 is a perspective view of the main part of the measuring apparatus according to an eighth embodiment of the present invention;
FIG. 17 is a perspective view of the main part of the measuring apparatus according to a ninth embodiment of the present invention;
FIGS. 18A, 18B, 18C and 18D are views each showing shapes of pressure distribution by a pair of feet of an erect standing subject and the change of the center of gravity, as output examples of analysis data for both footfall patterns by the above-described measuring apparatuses, respectively; and
FIG. 19 is a view showing the magnitude of distribution pressure at every segment point of the surface exerted by footfalls and the change of the center of gravity, as an output example of analysis data for footfall patterns by the above-described measuring apparatuses.

### PREFERRED EMBODIMENT OF THE INVENTION

Embodiments of the measuring apparatus for measuring reaction forces exerted by a subject against a support surface, according to the present invention will be explained with reference to the attached drawings, as follows.

### First Embodiment:

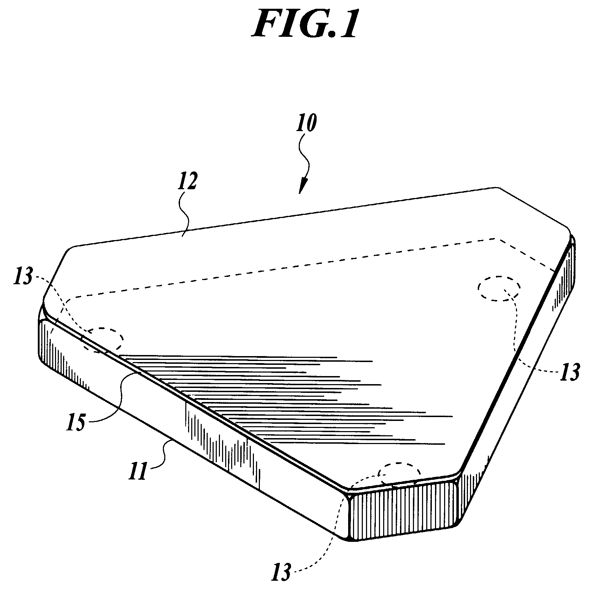

FIG. 1 is a perspective view of a first embodiment of the main part of the measuring apparatus for measuring reaction forces exerted by a subject against a support surface, according to the present invention.

In this figure, the reference numeral 10 denotes a forceplate which comprises a rigid platform 11 with a plate-like shape, and reaction force measuring members 13, e.g., load cells. The reference numeral 15 denotes a distribution shape sensing sheet having a thickness of about several mm or less, e.g., 0.2-2 mm.

In the embodiment, the forceplate 10 is used as a stabilometer. The platform 11 of the forceplate 10 is an approximate triangular shaped plate with a flat upper surface. The area of the flat upper surface 12 is large enough to place both feet of a subject thereon. On the entirety of the flat upper surface 12 of the forceplate 10, the distribution shape sensing sheet 15 is placed or adhered. Generally, the movement of the subject's body center of pressure is measured by placing the subject with bare feet on the distribution shape sensing sheet 15 during erect standing.

Under the platform 11, a load cell 13 is provided at each of the three peripheral corners thereof to support the platform horizontally.

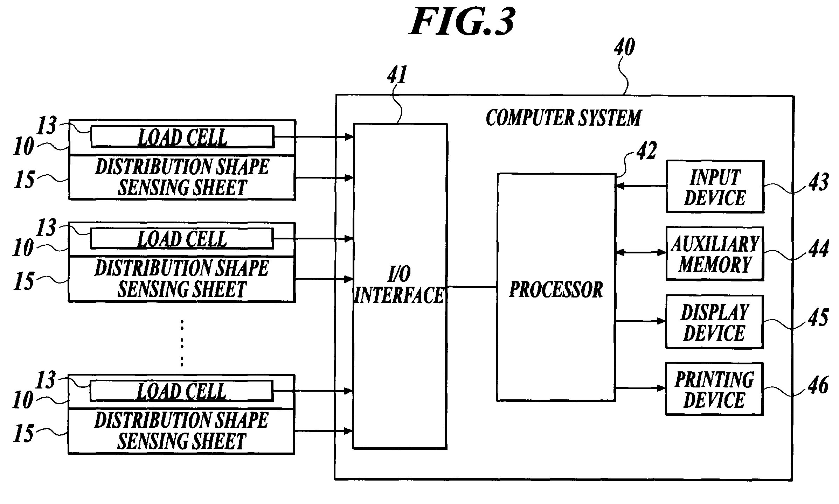

The three load cells 13, 13 and 13 detect a load exerted to the platform 11 in the vertical direction, i.e., Z-axis direction. Each of the three load cells 13, 13 and 13 detects the divided load exerted to the respective disposed position continuously and outputs to send the detected load information to an input-output interface 41 of a computer system 40 which is shown in FIG. 3.

The computer system 40 uses the detected load information from the three load cells 13, 13 and 13 to calculate the position of the subject's body center of reaction force, that is, a movement of the center of force, while the subject is erect standing on the platform 11.

As the distribution shape sensing sheet 15, one of various types of known sheet-like matrix-arrayed pressure sensors can be used. For example, it may comprise first and second flexible film substrates which are provided in parallel each other, a first large number of parallel electrodes formed on the inner surface of the first flexible film plate, a second large number of parallel electrodes which are perpendicular to the first large number of parallel electrodes and are formed on the inner surface of the second flexible film plate, and a resistive layer the resistance of which changes according to a pressure applied to the layer.

According to the measuring apparatus for measuring reaction forces of the first embodiment, it is possible not only to detect a shape of distribution of the load exerted to the upper surface of the platform by a distribution shape sensing sheet which is placed on an upper surface of the platform, but also to measure the magnitude of the load and the position of the center of pressure (COP) highly accurately by the load cells disposed under the platform at the same time because the load cells of the forceplate have a linear force/resistance characteristics and the output thereof is not affected by the subject's temperature.

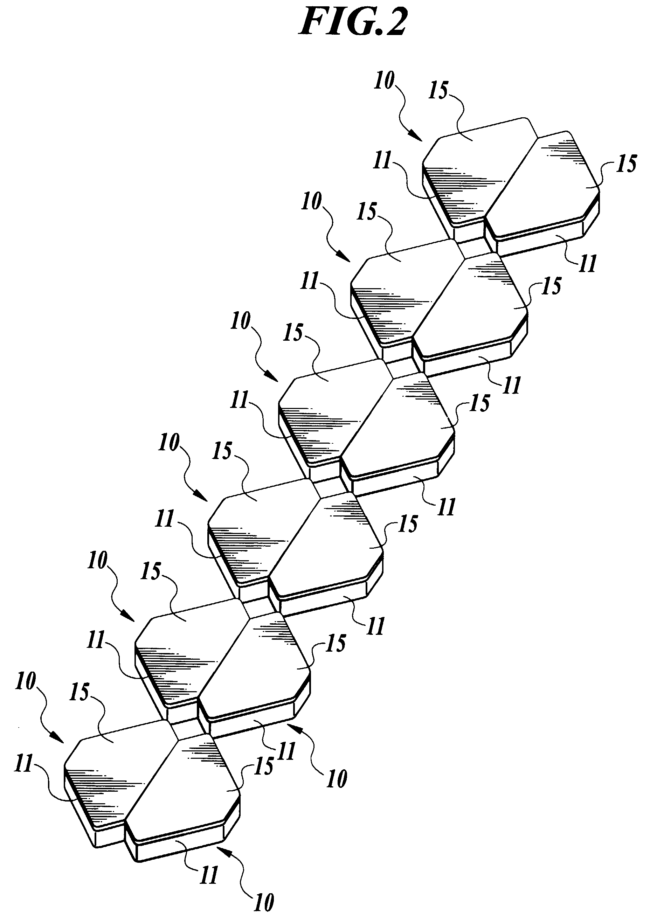

FIG. 2 is a perspective view of an embodiment of a walkway which is formed by a plurality of forceplates (gait plates) 10 each having a distribution shape sensing sheet 15 on the upper surface thereof, which are shown in FIG. 1. The walkway comprises a plurality of pairs of triangular forceplates 10 each having a distribution shape sensing sheet 15, which are disposed in a direction. Each pair is formed by bringing a side surface of one of the pair of triangular platforms 11 and 11 into contact with the corresponding side surface of the other of the pair. Then, a plurality of pairs are put in order in the extending direction of the contacting side surfaces, as shown in FIG. 2.

FIG. 3 is a block diagram of the measuring apparatus using the walkway shown in FIG. 2. In the figure, the computer system 40 comprises the input-output interface 41, a processor 42 which is a data analyzing member and comprises a CPU, RAM, ROM and the like, an input device 43, e.g., a keyboard, a control panel or the like, an auxiliary memory device 44, e.g., a hard disk drive, a floppy disk drive or the like, a display device 45, e.g., an LCD, a CRT display or the like, and a printing device 46, e.g., a printer or the like, which are output members for outputting analyzed data.

The output terminals of the load cells 13, 13 and 13 of each forceplate 10 and the output terminals of each distribution shape sensing sheet 15 are connected to the computer system 40 through the input-output interface 41.

When a subject walks on the walkway shown in FIG. 2, which comprises a plurality of pairs of triangular forceplates 10 each having a distribution shape sensing sheet 15, which are put in order in the direction, each of the three load cells 13, 13 and 13 of each forceplate 10 measures the respective divided one of the vertical reaction force (gravity component) exerted by the foot or feet of the subject against the upper surface of the platform 11, and the distribution shape sensing sheet 15 detects a shape of distribution (footprint) of the reaction forces exerted by the foot or feet of the subject against the forceplate 10 at the same time.

The measured data for the reaction force against the forceplate 10, from each load cell 13 and the sensed data for the shape of distribution of the reaction forces, from each distribution shape sensing sheet 15 are sent to the processor 42 of the computer system 40 through the input-output interface 41, as shown in FIG. 3. The processor 42 calculates to determine the position of the subject's body center of pressure, that is, a movement of the center of force, and the footprints, i.e., footfall patterns, successively, on the basis of these successively sent data.

The determined movement of position of the subject's body center of reaction force and footfall patterns are outputted to the display device 45 to display images thereof and also outputted to the printing device 46 to print them out.

As described above, according to the measurement apparatus of the embodiment of the invention, it is possible not only to detect the shape of distribution of the reaction forces exerted by the foot or feet of the subject against the platform 11 by the distribution shape sensing sheet 15 provided on the upper surface of the platform 11, but also to measure the sway of the subject's body center of pressure (COP) exerted by the subject against the platform 11 highly accurately by a plurality of load cells 13, 13 and 13 provided at the peripheral corners under the platform 11.

Accordingly, it is possible to obtain all of dynamic analysis data, i.e., dynamic loads (force), dynamic positions of the subject's body center of pressure, and dynamic distribution shapes by the foot or feet, during walking, extremely accurately at the same time, because the load cells have a linear force/resistance characteristics and the output thereof is not affected by the subject's temperature.

Because the measurement apparatus has a very simple and thin structure in which a plurality of load cells 13 are provided under the platform 11 and a thin distribution shape sensing sheet 15 is adhered to the upper surface of the platform 15, it is portable and enables measurement and analysis easily at any place.

### Second Embodiment:

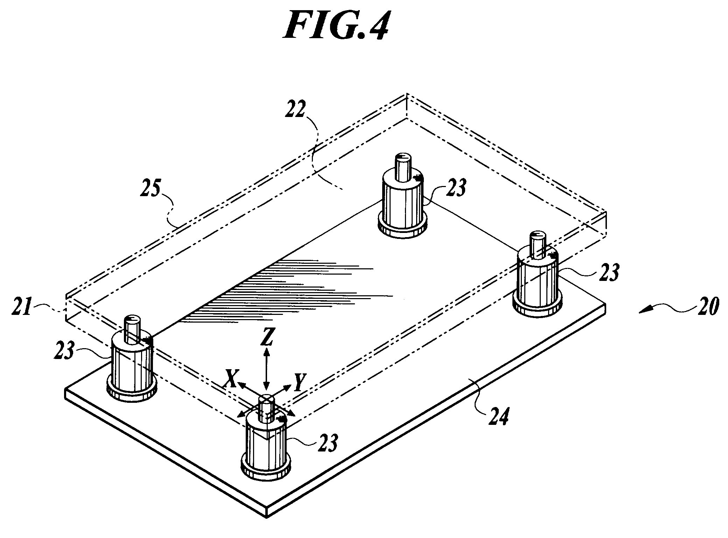

FIG. 4 is a perspective view of a second embodiment of the main part of the measuring apparatus for measuring reaction forces exerted by a subject against a support surface, according to the present invention.

In this figure, the reference numeral 20 denotes a forceplate which comprises a rigid platform 21 with a plate-like shape, and reaction force measuring members 23, i.e., three-force components detecting load cells. The reference numeral 25 denotes a rectangular distribution shape sensing sheet having almost the same inner structure as the distribution shape sensing sheet 15.

In the embodiment, the forceplate 20 is used as a reaction force measuring apparatus to a support surface.

The platform 21 of the forceplate 20 is an approximate rectangular shaped plate with a flat upper surface 22. The area of the flat upper surface 22 is large enough to place both feet of a subject thereon. On the entirety of the flat upper surface 22 of the forceplate 20, the distribution shape sensing sheet 25 is placed or adhered.

Under the rectangular shaped platform 21, a bottom plate 24 having approximately the same rectangular shape as the platform 21 is provided. At each of the four peripheral corners on the bottom plate 24, a three-force components detecting load cell is provided to support each of the four peripheral corners of the platform 21 horizontally.

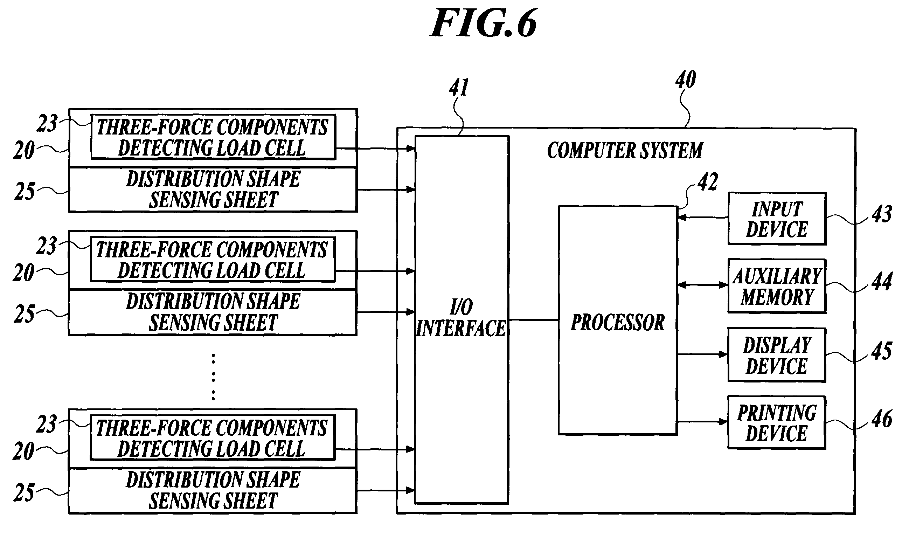

In each three-force components detecting load cell, X-axis and Y-axis force component detectors which detect force components of the reaction force exerted to a platform of a forceplate in the directions of X and Y axes perpendicular to each other, on the upper surface of the platform, respectively, and a Z-axis force component detector which detects the force component of the reaction force exerted to a platform in the direction of Z-axis perpendicular to the upper surface of the platform, are built-in. Each of the four three-force components detecting load cells 23, 23, 23 and 23 detects three-force components of the load exerted to the respective disposed position continuously and outputs to send the detected load information of the three-force components to the input-output interface 41 of the computer system 40 which is shown in FIG. 6.

The computer system 40 uses the detected load information of the three-force components and the information for the moment about each axis, from the four three-force components detecting load cells 23, 23, 23 and 23 to determine, for example, the subject's weight distribution during walking, acceleration force and decceleration force, locomotion of weight, torsion force, step length, when walking, gait velocity, gait stability and the like. Further, it is also possible to obtain analysis data for the subject's balancing during erect-standing, like the above-described stabilometer.

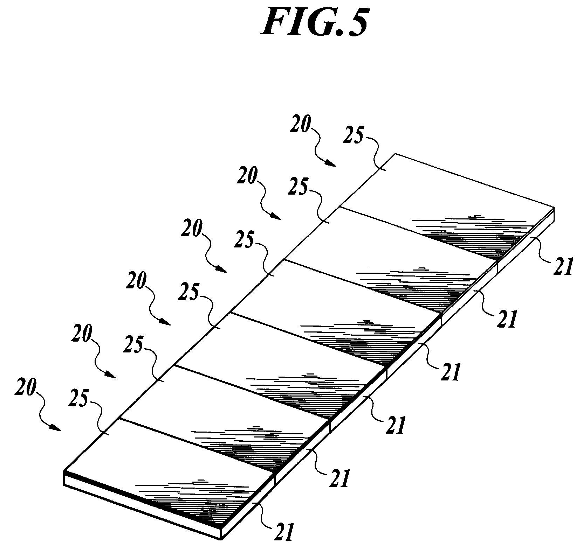

FIG. 5 is a perspective view of an embodiment of a walkway which is formed by a plurality of forceplates (gait plates) 20 each having a distribution shape sensing sheet 25 on the upper surface thereof, which are shown in FIG. 4. The walkway comprises a plurality of rectangular forceplates 20 each having a distribution shape sensing sheet 25, which are put in order in a direction, to contact the long side surfaces of each rectangular platform 21 with the long side surfaces of next one.

FIG. 6 is a block diagram of the measuring apparatus using the walkway shown in FIG. 5.

The output terminals of the four three-force components detecting load cells 23, 23, 23 and 23 of each forceplate 20 and the output terminals of each distribution shape sensing sheet 25 are connected to the computer system 40 through the input-output interface 41.

When a subject walks on the walkway shown in FIG. 5, which comprises a plurality of rectangular forceplates 20 each having a distribution shape sensing sheet 25, which are put in order in the direction, each of the four three-force components detecting load cells 23, 23, 23 and 23 of each forceplate 20 measures the force components in the directions of X, Y and Z axes, of the reaction force exerted to each three-force components detecting load cell 23, and the distribution shape sensing sheet 25 detects a shape of distribution of the reaction forces exerted by the foot or feet of the subject against the upper surface of the platform 21 at the same time.

The measured data for the X, Y and Z force components of the reaction force from each three-force components detecting load cell 23 and the sensed data for the shape of distribution of the reaction forces from each distribution shape sensing sheet 25 are sent to the processor 42 of the computer system 40 through the input-output interface 41, as shown in FIG. 6. The processor 42 calculates to determine desired information of the reaction forces exerted by the foot or feet of the subject against the upper surface of the platform 21 and the footprints, i.e., footfall patterns, successively, on the basis of these successively sent data.

The determined desired information of the reaction forces and footfall patterns are outputted to the display device 45 to display images thereof and also outputted to the printing device 46 to print them out.

As described above, according to the measurement apparatus of the embodiment of the invention, it is possible not only to obtain desired various types of information of the reaction forces exerted by the subject against the platform 21 highly accurately by each three-force components detecting load cell 23 provided at each peripheral corner under the platform 21, but also to detect the shape of distribution of the reaction forces exerted by the foot or feet of the subject against the platform 21 at the same time by the distribution shape sensing sheet 25 provided on the upper surface of the platform 21.

Accordingly, it is possible to obtain all of dynamic analysis data, i.e., dynamic loads (force), dynamic positions of the subject's body center of pressure, and dynamic distribution shapes by the foot or feet, during walking, extremely accurately at the same time, because the three-force components detecting load cells have a linear force/resistance characteristics and the output thereof is not affected by the subject's temperature.

Because the measurement apparatus has a very simple and thin structure in which a plurality of three-force components detecting load cell 23 are provided under the platform 21 and a thin distribution shape sensing sheet 25 is adhered to the upper surface of the platform 25, it is portable and enables measurement and analysis easily at any place.

### Third Embodiment:

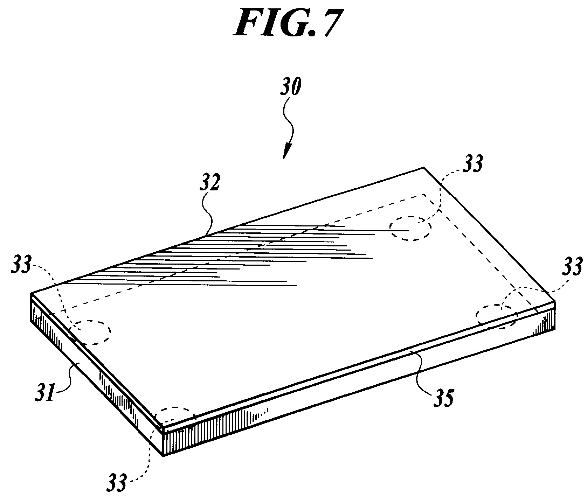

FIG. 7 is a perspective view of a third embodiment of a forceplate having a distribution shape sensing sheet, which is used in the measuring apparatus according to the present invention.

In this figure, the reference numeral 30 denotes a forceplate which comprises a rigid platform 31 with a rectangular plate-like shape, and four load cells 33, 33, 33 and 33 (reaction force measuring members) which are provided under the platform 31 at peripheral four corners thereof to support the platform 31 horizontally. The reference numeral 35 denotes a distribution shape sensing sheet which is placed or adhered on the entirety of the flat upper surface 32 of the platform 31. The distribution shape sensing sheet 35 and each load cell 33 have approximately the same structure and function as the above-described distribution shape sensing sheet 15 and each load cell 13 in the first embodiment, respectively.

The computer system 40 calculates to determine the position of the subject's body center of reaction force, that is, the center of sway, on the basis of the detected load information from the four load cells 33, 33, 33 and 33, while the subject is erect standing on the platform 31 of the forceplate 30.

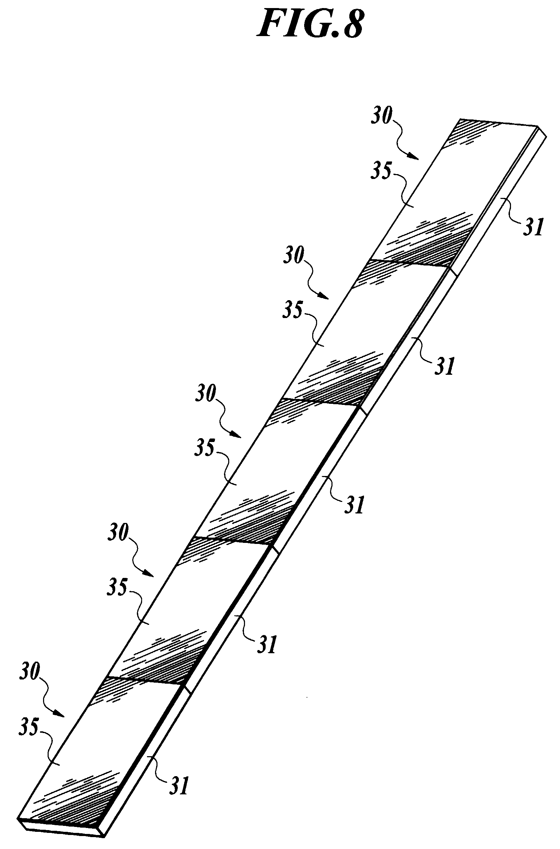

FIG. 8 is a perspective view of an embodiment of a walkway which is formed by a plurality of forceplates 30 each having a distribution shape sensing sheet 35 on the upper surface thereof, which are shown in FIG. 7. The walkway comprises a plurality of rectangular forceplates 30 each having a distribution shape sensing sheet 35, which are put in order in a direction, to contact the short side surfaces of each rectangular platform 31 with the short side surfaces of next one.

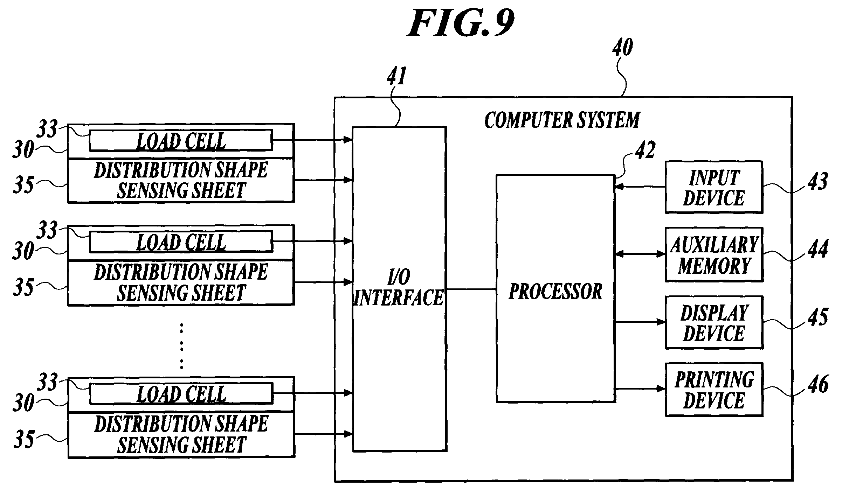

FIG. 9 is a block diagram of the measuring apparatus using the walkway shown in FIG. 8.

The output terminals of the four load cells 33, 33, 33 and 33 of each forceplate 30 and the output terminals of each distribution shape sensing sheet 35 are connected to the computer system 40 through the input-output interface 41.

When a subject walks on the walkway shown in FIG. 8, which comprises a plurality of rectangular forceplates 30 each having a distribution shape sensing sheet 35, which are put in order in the direction, each of the four load cells 33, 33, 33 and 33 of each forceplate 30 measures respective divided force of the reaction force exerted to the platform 31 in the vertical direction (gravity component) by the foot or feet of the subject, and the distribution shape sensing sheet 35 detects a shape of distribution of the reaction forces exerted by the foot or feet of the subject against the upper surface of the platform 31 at the same time.

The measured data for the reaction force against the forceplate 30, from each load cell 33 and the sensed data for the shape of distribution of the reaction forces, from each distribution shape sensing sheet 35 are sent to the processor 42 of the computer system 40 through the input-output interface 41, as shown in FIG. 9. The processor 42 calculates to determine the position of the subject's body center of reaction force, that is, center of sway, and the footprints, i.e., gait patterns, successively, on the basis of these successively sent data.

The determined movement of position of the subject's body center of reaction force and gait patterns are outputted to the display device 45 to display images thereof and also outputted to the printing device 46 to print them out.

As described above, according to the measurement apparatus of the embodiment of the invention, it is possible not only to measure the sway of the subject's COP exerted by the subject against the platform 31 highly accurately by four load cells 33, 33, 33 and 33 provided at the peripheral corners under the platform 31, but also to detect the shape of distribution of the reaction forces exerted by the foot or feet of the subject against the platform 31 at the same time by the distribution shape sensing sheet 35 provided on the upper surface of the platform 31 because the load cells have a linear force/resistance characteristics and the output thereof is not affected by the subject's temperature.

Accordingly, it is possible to obtain all of dynamic analysis data, i.e., dynamic loads (force), sway of the subject's body center of pressure, and dynamic distribution shapes by the foot or feet, during walking, extremely accurately at the same time.

Because the measurement apparatus has a very simple and thin structure in which a plurality of load cells 33 are provided under the platform 31 and a thin distribution shape sensing sheet 35 is adhered to the upper surface of the platform 35, it is portable and enables measurement and analysis easily at any place.

### [Example of Analysis Data Output]

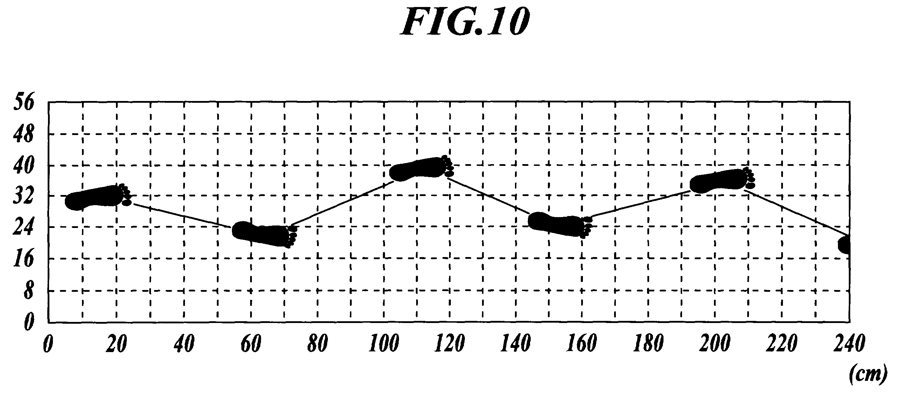

FIG. 10 is a view showing footprints with distribution shape and a locus of COP, as an output example of analysis data for gait patterns during walking by the measuring apparatus shown in FIG. 9.

Footprints (gait patterns during walking) and the locus of the center of pressure (center of sway) can be outputted as images by using the display device 45 and/or as a print by using the printing device 46, as shown in FIG. 10.

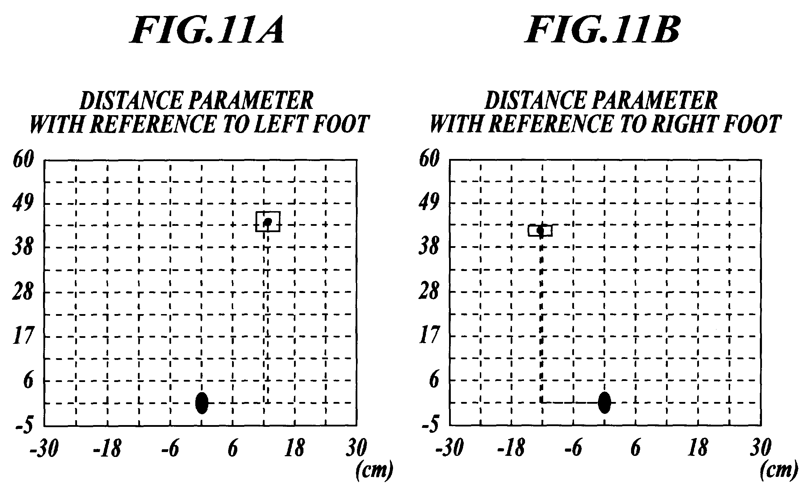

FIGS. 11A and 11B are views showing distance parameters with reference to right foot and left foot, respectively, as output examples of analysis data for gait patterns by the measuring apparatus shown in FIG. 9. In FIG. 11A, the elliptical black dot means the position of right foot which is the basis, and the rectangular framework including a dot therein means the variation region of existence of the center of pressure (COP) for the left foot landing, and the dot in the rectangular framework indicates the average position of the COP for the left foot landing. In FIG. 11B, the elliptical black dot means the position of left foot which is the basis, and the rectangular framework including a dot therein means the variation region of existence of COP for the right foot landing, and the dot in the rectangular framework indicates the average position of COP for the right foot landing. That is, FIGS. 11A and 11B show the variation region of existence of the COP for the landing left foot and landing right foot, and the distance thereof from the position of the right foot and of the left foot when the left foot and the right foot, of the subject lands on the basis of the right foot and of left foot, respectively. These variation can be outputted as images by using the display device 45 and/or as a print by using the printing device 46, as shown in these figures.

### Fourth Embodiment:

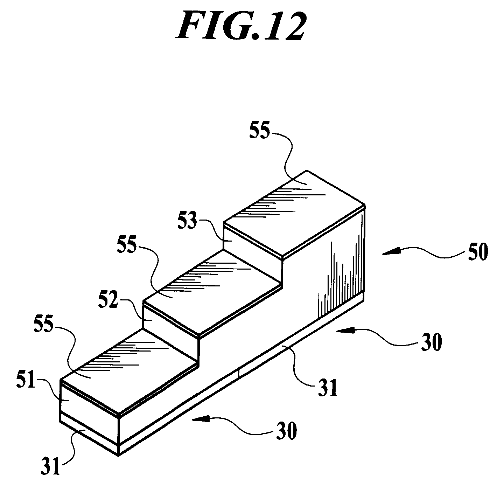

FIG. 12 is a perspective view of a fourth embodiment of the main part of the measuring apparatus for measuring reaction forces according to the present invention.

In this figure, the reference numeral 50 denotes a supplementary support which comprises two rectangular forceplates 30 and 30 placed in a line, stairs with three steps 51, 52 and 53 placed on the upper surface of the forceplates 30 and 30, and three distribution shape sensing sheets 55, 55 and 55 which are placed or adhered on the entirety of the upper surface of the steps 51, 52 and 53 of the stairs, respectively. Each forceplate 30 comprises a rigid rectangular platform 31 and four load cells 33, 33, 33 and 33 which are provided under the platform 31 at peripheral four corners thereof to support the platform 31 horizontally, as shown in FIG. 7. However, each forceplate has no distribution shape sensing sheet on the upper surface thereof. The distribution shape sensing sheets 35 which are provided on the upper surfaces of steps 51, 52 and 53 have approximately the same structure and function as the above-described distribution shape sensing sheet 15, 25 or 35 in the prior embodiment.

According to the measurement apparatus of the embodiment, it is possible to obtain all of dynamic analysis data, i.e., dynamic loads (force), dynamic positions of the subject's body center of pressure, and dynamic distribution shapes by the foot or feet, during ascending or descending the stairs, extremely accurately at the same time because the load cells have a linear force/resistance characteristics and the output thereof is not affected by the subject's temperature.

### Fifth Embodiment:

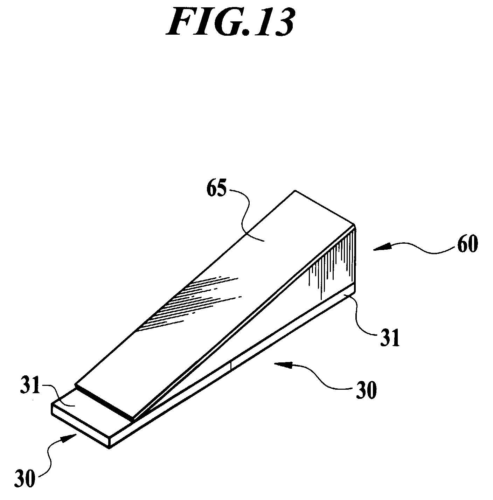

FIG. 13 is a perspective view of a fifth embodiment of the main part of the measuring apparatus for measuring reaction forces according to the present invention.

In this figure, the reference numeral 60 denotes a supplementary support which comprises two rectangular forceplates 30 and 30 which are almost the same as the ones 30 and 30 in the fourth embodiment and are placed in a line, a slope with an inclined surface placed on the upper surface of the platforms 31 and 31 of the forceplates 30 and 30, and a distribution shape sensing sheet 65 which is placed or adhered on the entirety of the inclined surface of the slope. Each forceplate 30 has no distribution shape sensing sheet. The distribution shape sensing sheets 65 provided on the inclined surface has approximately the same structure and function as the above-described distribution shape sensing sheet 15, 25, 35 or 55 in the prior embodiment.

According to the measurement apparatus of the embodiment, it is possible to obtain all of dynamic analysis data, i.e., dynamic loads (force), dynamic positions of the subject's body center of pressure, and dynamic distribution shapes by the foot or feet, during ascending or descending the slope, extremely accurately at the same time because the load cells have a linear force/resistance characteristics and the output thereof is not affected by the subject's temperature.

### Sixth Embodiment:

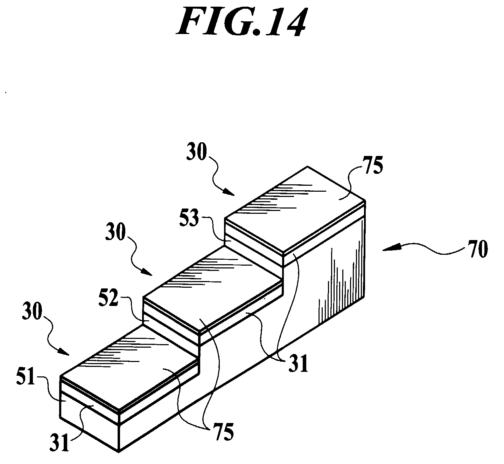

FIG. 14 is a perspective view of a sixth embodiment of the main part of the measuring apparatus for measuring reaction forces according to the present invention.

In this figure, the reference numeral 70 denotes a supplementary support which comprises stairs with three steps 51, 52 and 53, three rectangular forceplates 30, 30 and 30 placed or adhered on upper surfaces of the three steps 51, 52 and 53 of the stairs, respectively, and three distribution shape sensing sheets 75, 75 and 75 which are placed or adhered on the entirety of the upper surface of the forceplates 30, 30 and 30, respectively. Each forceplate 30 comprises a rigid rectangular platform 31 and four load cells 33, 33, 33 and 33 which are provided under the platform 31 and on the upper surface of the corresponding step 51, 52 or 53, at peripheral four corners thereof to support the platform 31 horizontally. On the upper surface of each platform 31, a distribution shape sensing sheet 75 is provided. Each distribution shape sensing sheets 75 has approximately the same structure and function as the above-described distribution shape sensing sheet 15, 25, 35, 55 or 65 in the prior embodiment.

According to the measurement apparatus of the embodiment, it is possible to obtain all of dynamic analysis data, i.e., dynamic loads (force), dynamic positions of the subject's body center of pressure, dynamic distribution shapes by the foot or feet, and also magnitude of the distribution pressure, during ascending or descending the stairs of the support 70, extremely accurately at the same time because the load cells have a linear force/resistance characteristics and the output thereof is not affected by the subject's temperature.

### Seventh Embodiment:

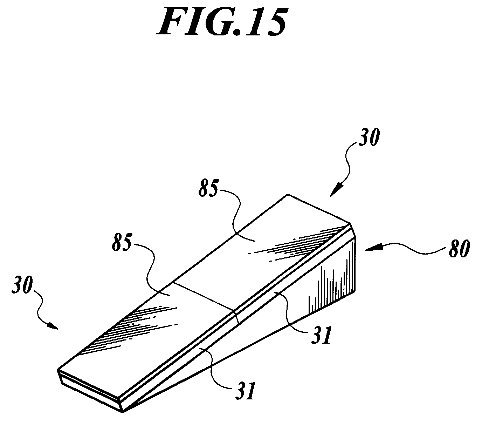

FIG. 15 is a perspective view of a seventh embodiment of the main part of the measuring apparatus for measuring reaction forces according to the present invention.

In this figure, the reference numeral 80 denotes a supplementary support which comprises a slope with an inclined surface, two rectangular forceplates 30 and 30 placed on the upper surface of the inclined surface of the support 80, and distribution shape sensing sheets 85 and 85 which are placed or adhered on the entirety of two rectangular forceplates 30 and 30, respectively. Each forceplate 30 comprises a rigid rectangular platform 31 and four load cells 33, 33, 33 and 33 which are provided under the platform 31 and on the upper surface of the inclined surface of the support 80, at peripheral four corners thereof to support the platform 31 in parallel with the inclined surface. The distribution shape sensing sheets 85 provided on the inclined surface has approximately the same structure and function as the above-described distribution shape sensing sheet 15, 25, 35, 55, 65 or 75 in the prior embodiment.

According to the measurement apparatus of the embodiment, it is possible to obtain all of dynamic analysis data, i.e., dynamic loads (force), dynamic positions of the subject's body center of pressure, dynamic distribution shapes by the foot or feet, and also magnitude of the distribution pressure, during ascending or descending the slope, extremely accurately at the same time because the load cells have a linear force/resistance characteristics and the output thereof is not affected by the subject's temperature.

### Eighth Embodiment:

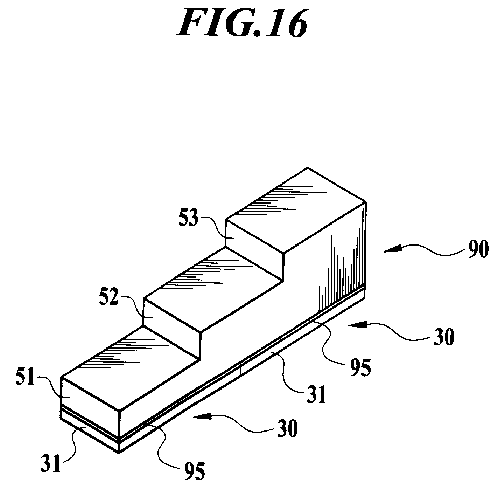

FIG. 16 is a perspective view of an eighth embodiment of the main part of the measuring apparatus for measuring reaction forces according to the present invention.

In this figure, the reference numeral 90 denotes a supplementary support which comprises two rectangular forceplates 30 and 30 placed in a line, two distribution shape sensing sheets 95 and 95 which are placed or adhered on the entirety of the upper surface of the respective rectangular forceplates 30 and 30, and stairs with three steps 51, 52 and 53 placed on the upper surface of the two distribution shape sensing sheets 95 and 95. Each forceplate 30 has approximately the same structure and function as the one in the fourth or fifth embodiment. Each distribution shape sensing sheets 95 has approximately the same structure and function as the above-described distribution shape sensing sheet in the prior embodiment.

### Ninth Embodiment:

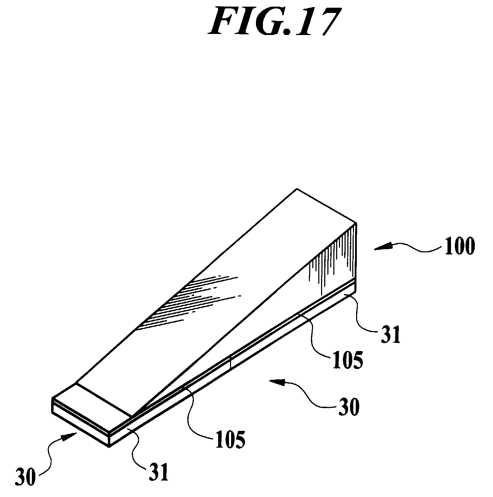

FIG. 17 is a perspective view of a ninth embodiment of the main part of the measuring apparatus for measuring reaction forces according to the present invention.

In this figure, the reference numeral 100 denotes a supplementary support which comprises two rectangular forceplates 30 and 30 placed in a line, two distribution shape sensing sheets 105 and 105 which are placed or adhered on the entirety of the upper surface of the respective rectangular forceplates 30 and 30, and a slope with an inclined surface. Each forceplate 30 has approximately the same structure and function as the one in the fourth or fifth embodiment. Each distribution shape sensing sheets 105 has approximately the same structure and function as the above-described distribution shape sensing sheet in the prior embodiment.

### Another Embodiment:

In each of the above-described the first to ninth embodiments, although only a distribution shape sensing sheet 15, 25, 35, 55, 65, 75, 85, 95 or 105 is used as a sheet for sensing a shape of distribution of the reaction forces exerted by the subject against the platform, a distribution pressure measuring sheet which can sense not only the shape of distribution of the reaction forces but also the magnitude of distribution pressure at each point in a detection region.

For example, one of the distribution pressure measuring sheets comprises a pressure sensitive conductive material thin layer formed between a pair of parallel striped electrodes layers which are used in the above-described distribution shape sensing sheet. The pressure sensitive conductive layer is made of, for example, a pressure sensitive conductive rubber which comprises silicone rubber and electroconductive particles dispersed therein. When an applied pressure compresses a portion of the pressure sensitive conductive layer, the electrical resistance thereof is reduced. That is, use of a pressure sensitive conductive layer enables transduction from a pressure to a resistance value.

In the matrix composed of the pair of parallel striped electrodes layers, a voltage is applied to the striped electrodes of one of the electrodes layers in order and the corresponding resistive value is measured through the striped electrodes of the other of the electrodes layers in order. Thus, the resistive value of each intersection of the pair of striped electrodes of the two electrode layers is measured in order by scanning.

According to the measurement apparatus using such a distribution pressure measuring sheet instead of the distribution shape sensing sheets 15, 25, 35, 55, 65, 75, 85, 95 and 105 in the first to ninth embodiments, it is possible to obtain highly accurate analysis data on the basis of the measured data for the reaction force from the load cells or three-force components detecting load cells and the measured data for the distribution pressure. That is, it is possible to obtain all of analysis data, i.e., loads (force), positions of the subject's body center of pressure, distribution shapes by the foot or feet, and all for the distribution pressure including the magnitude thereof, extremely accurately at the same time because the load cells have a linear force/resistance characteristics and the output thereof is not affected by the subject's temperature.

### [Example of Analysis Data Output]

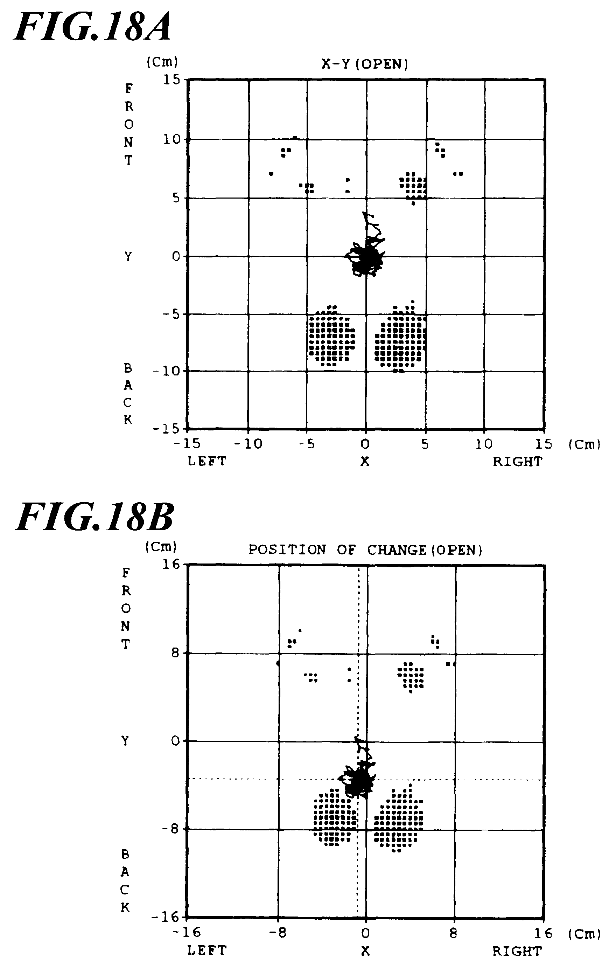

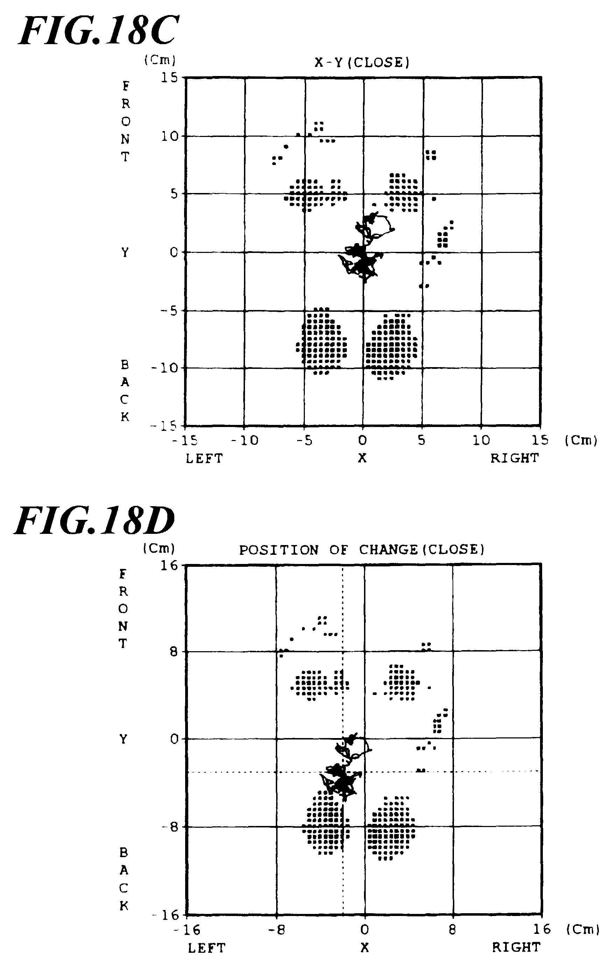

FIGS. 18A to 18D are views showing footprints of pressure distribution of the reaction forces and a locus of the COP, as an output example of analysis data for footprints, during erect standing, by one of the measuring apparatuses. FIG. 18A shows one when the subject is standing with eyes open and the COP is put on the origin of the apparatus, FIG. 18B shows the position of the center of sway with respect to the origin of the apparatus when the subject is standing with eyes open and the feet are put on the predetermined positions on the distribution shape sensing sheet, FIG. 18C shows one when the subject is standing with eyes close and the COP is put on the origin of the apparatus, and FIG. 18D shows the position of the center of sway with respect to the origin of the apparatus when the subject is standing with eyes close and the feet are put on the predetermined positions on the distribution shape sensing sheet.

Thus, footprints of both feet and the sway of COP, during erect standing can be outputted as images by using the display device 45 at the same time. According to the measuring apparatus of the above embodiments, it is possible to assess the physical function of the subject by using a picture on the display as it is, during a test or training, and it is valuable for training.

Such footprints of both feet and the movement of the center of pressure thereof can be outputted as a print by the printing device 46.

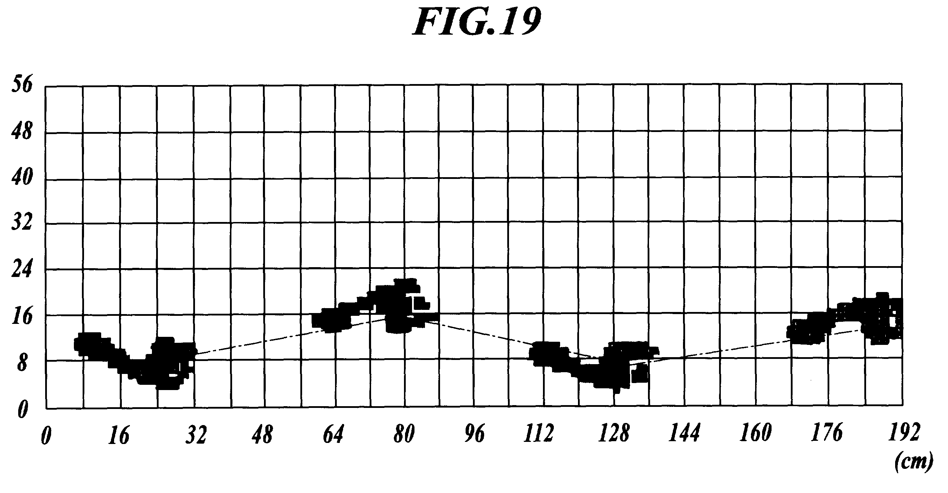

FIG. 19 is a view showing gait patterns during walking, each of which comprises a large number of divided sensor elements indicating the magnitude of distribution pressure at each divided element on the surface exerted by footfalls and showing the locus of COP, as an output example of analysis data for gait patterns by the above-described measuring apparatuses using a distribution pressure measuring sheet.

Footprints of both feet, the state of magnitude of distribution pressure at each divided element in the footprint, and the locus of COP, during walking can be outputted as images by using the display device 45 at the same time, as shown in FIG. 19. According to the measuring apparatus of the embodiments, it is possible to assess the physical function of a subject by using a picture on the display as it is, during a test or training, and it is further valuable for training.

Such footprints of both feet, the state of magnitude of distribution pressure at each divided sensor element in the footprint, and the locus of COP can be outputted as a print by the printing device 46.

Such a view showing gait patterns and locus of COP during walking can be outputted also for the measuring apparatus using a distribution shape sensing sheet.

Although some embodiments of the invention have been explained as described above, it should also be understood that the present invention is not limited to the embodiments and that various changes and modifications may be made to the invention without departing from the gist thereof.

For example, in the above-described embodiments, although only data analysis and output, during walking is explained, the present invention can be used for data analysis and output, during another postural change, e.g., rising from a seated surface.

In the above-described embodiments, although only a plurality of forceplates put in order are used as a long walkway, it may use a single long walkway to obtain data analysis and output during a desired posture.

The type or shape of the support 50, 60, 70, 80, 90 or 100 for test is optional. For example, an examination support having an upper curved surface may be also used. It is of course that the concrete structure thereof may be also changed properly.

The entire disclosure of Japanese Patent Application Nos. Tokugan Hei-11-222775 which was filed on August 5, 2000, including specification, claims, drawings and summary are incorporated herein by reference in its entirety.

## Claims

1. A measuring apparatus for measuring reaction forces exerted by a subject against a support surface, comprising:
a forceplate comprising a platform with the support surface for placing the subject thereon, and a reaction force measuring member disposed under the platform, for sensing the reaction forces exerted by the subject against the platform,
a distribution shape sensing sheet for sensing a shape of distribution of the reaction forces exerted by the subject against the platform, or a distribution pressure measuring sheet for sensing distribution pressure of the reaction forces, which is placed on an upper surface of the platform, and
an output member for outputting analysis data on the basis of data of the reaction forces sensed by the reaction force measuring member and data of the shape of distribution sensed by the distribution shape sensing sheet or of the distribution pressure sensed by the distribution pressure measuring sheet.

2. The measuring apparatus as claimed in claim 1, further comprising a supplementary support having stairs, an upper inclined surface or an upper curved surface, on an upper surface of which the distribution shape sensing sheet or the distribution pressure measuring sheet is placed.

3. A measuring apparatus for measuring reaction forces exerted by a subject against a support surface, comprising:
a walkway comprising a set of a plurality of gait plates arranged in order, each of the gait plates comprising: a forceplate having a platform with the support surface for placing the subject thereon, and a reaction force measuring member disposed under the platform, for sensing the reaction forces exerted by the subject against the platform; and a distribution shape sensing sheet for sensing a shape of distribution of the reaction forces exerted by the subject against the platform, or a distribution pressure measuring sheet for sensing distribution pressure of the reaction forces, which is placed on an upper surface of the platform; and
an output member for outputting analysis data on the basis of data of the reaction forces sensed by the reaction force measuring member and data of the shape of distribution sensed by the distribution shape sensing sheet or of the distribution pressure sensed by the distribution pressure measuring sheet, of the gait plates.

4. A measuring apparatus for measuring reaction forces exerted by a subject against a support surface, comprising:
a supplementary support which comprises: a forceplate having a rigid platform and three or more load cells provided under the platform at peripheral corners thereof to support the platform; a body having at least one of stairs, a slope and a curved surface, placed on the upper surface of the platform; and a distribution shape sensing sheet for sensing a shape of distribution of the reaction forces exerted by the subject against the platform, or a distribution pressure measuring sheet for sensing distribution pressure of the reaction forces, which is placed on an upper surface of the body, and
an output member for outputting analysis data on the basis of data of the reaction forces sensed by the load cells and data of the shape of distribution sensed by the distribution shape sensing sheet or of the distribution pressure sensed by the distribution pressure measuring sheet.

5. The measuring apparatus as claimed in claim 1, 2 or 4, wherein the output member is a display device which displays: a shape of distribution of the reaction forces on the basis of the data sensed by the distribution shape sensing sheet or a state of distribution pressure on the distribution pressure measuring sheet on the basis of data sensed by the distribution pressure measuring sheet; and a center of pressure on the basis of data analysis of the reaction forces sensed by the reaction force measuring member.

6. The measuring apparatus as claimed in claim 3, wherein the output member is a display device which displays: a locus of a shape of distribution of the reaction forces on the basis of the data sensed by the distribution shape sensing sheets of the gait plates or a locus of a state of distribution pressure on the distribution pressure measuring sheets on the basis of data sensed by the distribution pressure measuring sheets; and a locus of a center of pressure on the basis of data analysis of the reaction forces sensed by the reaction force measuring members.

7. A measuring apparatus for measuring reaction forces exerted by a subject against a support surface, comprising:
a forceplate comprising a rigid platform with the support surface for placing the subject thereon, and a plurality of reaction force measuring members disposed at peripheral positions of and under the platform, for detecting loads exerted to the respective disposed positions on the platform continuously and outputting load information signals on the basis of the detected loads,
a distribution shape sensing sheet for sensing a shape of distribution of the reaction forces exerted by the subject against the platform, or a distribution pressure measuring sheet for sensing distribution pressure of the reaction forces, which is placed on an upper surface of the platform,
a processor for calculating to determine a total amount of reaction force exerted to the platform by the subject, a position of the center of reaction force and a shape of distribution or distribution pressure, of the reaction force, on the basis of load information signals from the reaction force measuring members and shape of distribution signals from the distribution shape sensing sheet or distribution pressure signals from the distribution pressure measuring sheet, respectively, and
an output member to enable outputting the total amount of reaction force exerted to the platform by the subject, the position of the center of reaction force and the shape of distribution or the distribution pressure, of the reaction force, determined by the processor.

8. The measuring apparatus as claimed in claim 7; wherein the plurality of reaction force measuring members comprise load cells or three-force components detecting load cells, not less than 3.

9. A measuring method for measuring reaction forces exerted by a subject against a support surface, comprising the steps of:
preparing a forceplate comprising a rigid platform with the support surface for thereon, and a plurality of reaction force measuring members disposed at peripheral positions of and under the platform, to detect loads exerted to the respective disposed positions on the platform continuously and to output load information signals on the basis of the detected loads,
placing a distribution shape sensing sheet for sensing a shape of distribution of the reaction forces or a distribution pressure measuring sheet for measuring distribution pressure of the reaction forces on an upper surface of the platform,
placing a subject on an upper surface of the distribution shape sensing sheet or the distribution pressure measuring sheet,
calculating to determine a total amount of reaction force exerted to the platform by the subject, a position of the center of reaction force and a shape of distribution of the reaction force or distribution pressure of the reaction force, by a processor, on the basis of load information signals from the reaction force measuring members and shape of distribution signals from the distribution shape sensing sheet or distribution pressure signals from the distribution pressure measuring sheet, respectively, and
outputting the total amount of reaction force, the position of the center of reaction force exerted to the platform by the subject and the shape of distribution or the distribution pressure, of the reaction force, determined by the processor.

10. The measuring method as claimed in claim 9, wherein the step of outputting comprises displaying the shape of distribution of the reaction forces on the basis of the data sensed by the distribution shape sensing sheet or a state of distribution pressure on the distribution pressure measuring sheet on the basis of data sensed by the distribution pressure measuring sheet; and displaying the center of pressure on the basis of data analysis of the reaction forces sensed by the reaction force measuring member, at the same time.

11. A measuring method for measuring reaction forces exerted by a subject against a support surface, comprising the steps of:
placing the subject on a walkway comprising a set of a plurality of gait plates arranged in order, each of the gait plates comprising: a forceplate having a platform with the support surface for placing the subject thereon, and a reaction force measuring member disposed under the platform, for sensing the reaction forces exerted by the subject against the platform; and a distribution shape sensing sheet for sensing a shape of distribution of the reaction forces exerted by the subject against the platform, or a distribution pressure measuring sheet for sensing distribution pressure of the reaction forces, which is placed on an upper surface of the platform; and
sensing a shape of distribution of the reaction forces exerted by the subject against the platform, by using a distribution shape sensing sheets of the gait plates, or sensing distribution pressure of the reaction forces by using a distribution pressure measuring sheet of the gait plates, which are placed on upper surfaces of the platforms of the gait plates,
calculating to determine a total amount of reaction force exerted to each platform by the subject, a position of the center of reaction force and shapes of distribution of the reaction force or distribution pressure of the reaction force, by a processor, on the basis of load information signals from the reaction force measuring members and shape of distribution signals from the distribution shape sensing sheets or distribution pressure signals from the distribution pressure measuring sheets, respectively, and
outputting the total amount of reaction force, the position of the center of reaction force exerted to the platform by the subject and the shape of distribution or the distribution pressure, of the reaction force, on each gait plate, determined by the processor.

12. The measuring method as claimed in claim 11, wherein the step of outputting comprises displaying a locus of shape of distribution of the reaction forces on the basis of the data sensed by the distribution shape sensing sheets of the gait plates or a locus of a state of distribution pressure on the distribution pressure measuring sheets on the basis of data sensed by the distribution pressure measuring sheets; and a locus of a center of pressure on the basis of data analysis of the reaction forces sensed by the reaction force measuring members, at the same time.
